# EUROPEAN PATENT APPLICATION

(11) **EP 4 523 640 A1**
(43) Date of publication of application: **19.03.2025**
(21) Application number: 24198225.5
(22) Date of filing: 03.09.2024
(51) Int. Cl.: A61B 17/54, A61B 90/00

(54) **CALLUS REMOVER WITH A SAFETY FIXING SYSTEM**

(30) Priority: 14.09.2023 ES 202331624 U
(71) Applicant: Rob Jor Bebe S.L., 01320 Oyon Alava (ES)
(72) Inventor: CASTROVIEJO CESPEDES, JUAN CARLOS, 26003 Logroño (La Rioja) (ES)
(74) Representative: Maslanka Kubik, Dorota Irena

(57) **Abstract**

The subject invention refers to a callus remover with a safety fixing system, comprising at least: a handle (16); a head (2) after the handle (16); an upper part (1); and a cutting blade (3) which is secured between the head (2) and the upper part (1); with the particularity that the safety fixing system has the following features: the upper part (1) and the head (2) are attached by means of a displaceable hinge-type articulated attachment (4) which configures a relative displacement of the upper part (1) with respect to the head (2); and a relative rotation between the upper part (1) and the head (2) in at least one location included along the relative displacement of the upper part (1) with respect to the head (2).

## Description

### TECHNICAL FIELD

The present invention is comprised within the personal care and hygiene sector, particularly in the sector of instruments and apparatus for cutting calluses.

### BACKGROUND OF THE INVENTION

Today, callus removers are widely known as personal hygiene instruments used for cutting calluses, comprising a removable cutting blade between two separable parts, which must be replaced with a new cutting blade after a certain period of use.

The replacement of the cutting blade comprises two steps: an opening step in which the two parts of the callus remover are separated from one another to remove the cutting blade to be replaced; and a closing step after placement of the new cutting blade, in which the two parts of the callus remover are attached and fixed to one another again, securing the new cutting blade.

There are callus removers in which, to be able to access the cutting blade and take it out, the two parts are disassembled, being completely separated from one another. The fixing systems commonly used to fix the two parts of the callus remover in the closing step are screw type systems, pressure systems or other similar systems. The use of these fixing systems involves safety issues when replacing the cutting blade, as they require the use of both hands, together with performing manual operations which can cause unwanted cuts in the user.

Therefore, a callus remover that improves the handling thereof, primarily in relation to the safety and ease in the operation for replacing the cutting blade, is necessary.

### DESCRIPTION OF THE INVENTION

The callus remover with a safety fixing system proposed by the invention is configured as a remarkable novelty within its field of application, as, due to its implementation, the objectives indicated below are achieved, and the characterizing details which make it possible and distinguish it are suitably described in the final claims attached to the present description.

The callus remover object of the present invention proposes the replacement of the fixing systems commonly used in the prior art with a simple fixing system that improves the safety in the process of removing and placing the cutting blade, as the entire operation related to the opening and closing of the callus remover can be performed with a single hand, which furthermore is far enough away from the cutting areas of the cutting blade, avoiding the possibility of possible unwanted cuts.

Additionally, the fixing system improves the ease of the operation of changing and replacing the cutting blade.

To achieve the above objectives, the callus remover object of the present invention comprises at least: a handle; a head after the handle; an upper part; a cutting blade which is secured between the head and the upper part; and a fixing system with the following features:
The upper part and the head are attached by means of a displaceable hinge-type articulated attachment which allows the displacement of the upper part along the head and, in at least one position of the length covered by the displacement, the relative rotation of the upper part with respect to the head. In this case, the term "position" is synonymous with location.

In this way, when the upper part is in a closed position, in which it is closed on the head securing the cutting blade between both, if the user wants to access the cutting blade, he/she must simply displace the upper part from the closed position to an open position, in which the upper part can rotate with respect to the head, releasing the cutting blade for its replacement.

Preferably, when the callus remover is closed, the cutting blade is curved between the upper part and the head. The cutting blade tends to recover its original flat shape, but the upper part prevents this, so the cutting blade generates a thrust on the upper part. This thrust generates sufficient friction between the upper part and the head to ensure its fixing in the closed position. In this case, if the user wants to access the cutting blade, he/she must simply press the upper part against the head and displace the upper part from the closed position to the open position.

Due to the simplicity of the maneuver for displacement of the upper part, said maneuver can be performed by the user, for example, by means of using his/her thumb gripping the callus remover, positioned on the upper part, and the upper part can be displaced along the head and placed in the closed position or in the open position, as desired, with the user's other hand being free to be able to safely handle the cutting blade.

The displaceable hinge-type articulated attachment comprises at least one movement means made up of the following components:
- a guiding component in the head, which allows the guided longitudinal displacement of the upper part; and
- a guided component in the upper part, such that the guided component and, accordingly, the upper part, can be displaced in a guided manner along the head.

The respective morphologies of the guiding component and guided component are morphologies which, together, allow the particular kinematics of the components. Namely, said morphologies allow the displacement of the guided component along the guiding component (and, accordingly, allow the longitudinal displacement of the upper part with respect to the head), and in at least one position (synonymous with location) of the length covered by the displacement, allow the relative rotation of the guided component with respect to the guiding component (and, accordingly, the relative rotation of the upper part with respect to the head).

In a particular embodiment, the guiding component of the movement means is a slot located longitudinally in the head, and the guided component of the movement means is a protrusion located in the upper part, which is at least partially inserted into the slot.

In one embodiment, the particular kinematics of the movement means described above is achieved as a result of the following morphologies of the slot and the protrusion:
- The slot comprises two areas: a wide area and a narrow area. Preferably, the slot has a substantially circular shape in the wide area. Preferably, the narrow area of the slot is defined between two parallel planes (for example, it has a substantially rectangular shape) separated from one another by a distance referred to as "slot thickness", with the slot thickness being smaller than the recess of the wide area (smaller than the diameter of the wide area, in the event that it is circular).
- The protrusion has a substantially rectangular cross-section morphology, with a larger dimension referred to as "length of the protrusion" and a smaller dimension referred to as "thickness of the protrusion". For example, according to a particular embodiment, the protrusion is defined by four perimeter surfaces: two opposite surfaces that are flat and parallel to one another, and two other surfaces opposite one another having a curved shape. The distance between the curved surfaces is greater than the distance between the parallel flat surfaces.

The thickness of the protrusion coincides with the thickness of the narrow area of the slot. Namely, the term "coincides" in relation to the thickness of the protrusion, means that the thickness of the protrusion is a little smaller than the thickness of the narrow area of the slot, such that there is a little clearance which allows the sliding of the protrusion along the slot, but not the rotation thereof.

The length of the protrusion coincides with the recess of the wide area of the slot (coincides with the diameter of the wide area, in the event that it is circular). Namely, the term "coincides" in relation to the length of the protrusion, means that the length of the protrusion is a little smaller than the recess of the wide area of the slot, such that there is a little clearance which allows the rotation of the protrusion in said recess.

As described, when the protrusion is located in the narrow area of the slot, the protrusion and, accordingly, the upper part, can slide along the slot, but cannot rotate with respect to the head of the callus remover; and when the protrusion is located in the wide area of the slot, the protrusion can rotate, and, accordingly, the upper part can rotate with respect to the head of the callus remover.

When the expression "substantially circular" is used herein in reference to a part of the invention (where "part" can refer to a component or area of a component), it is to be understood that the mentioned part has a circular or approximately circular shape, without departing from the functions of said part in the present invention. Similarly, when the expression "substantially rectangular" is used in reference to a part of the invention, it is to be understood that the mentioned part has a rectangular or approximately rectangular shape, without departing from the functions of said part in the present invention.

In a possible embodiment, the displaceable hinge-type articulated attachment comprises two movement means such as those defined above, one on each side of the callus remover.

In another possible embodiment, the displaceable hinge-type articulated attachment comprises:
- Main movement means located on one side of the callus remover, with the same characteristics as the movement means defined above.
- Complementary movement means located on the opposite side of the callus remover in turn comprising:
   ▪ a guiding component in the head, which allows the guided longitudinal displacement of the upper part;
   ▪ a guided component in the upper part, such that the guided component and, accordingly, the upper part, can be displaced in a guided manner along the head.

Unlike the main movement means, in this case, the respective morphologies of the guiding component and guided component of the complementary movement means do not have the function of preventing the rotation of the guided component in any area of the guiding component, but rather are simply limited to performing a guiding function, obviously without preventing the particular kinematics of the main movement means.

In one embodiment:
- The guiding component of the complementary movement means is a slot located longitudinally in the head. Preferably, the slot is defined between two parallel planes (for example, it has a substantially rectangular shape) separated from one another by a distance referred to as "slot thickness".
- The guided component of the complementary movement means is a protrusion located in the upper part, which is at least partially inserted into the respective slot, with the particularity that the size of the protrusion is smaller than the slot thickness. Taking as a reference a cross-section of the protrusion, the size of the protrusion refers to the distance between the two points of the contour of said section that are farthest away from one another. The protrusion can have different shapes, for example, it can have a rectangular cross-section, a circular cross-section, a pentagonal cross-section, etc.

In a possible embodiment, the callus remover comprises stops limiting the longitudinal displacement of the upper part with respect to the head in one direction and in the opposite direction, respectively. According to one embodiment, the callus remover comprises:
- a front stop limiting the forward displacement of the upper part with respect to the head, beyond the closed position;
- a rear stop limiting the backward displacement of the upper part with respect to the head, beyond the open position.

It is to be understood herein that the "forward" displacement of the upper part is that which is directed towards the closed position, and it is to be understood herein that the "backward" displacement of the upper part is that which is directed towards the open position.

In a possible embodiment, the callus remover comprises at least one tab-housing assembly made up of a tab and a housing. The tab is located in one of the following components of the callus remover: the upper part or the head. The housing is located in the component opposite the one in which the tab is located, such that, in the closed position of the upper part, the tab is inserted into the housing. In a particular example, the callus remover comprises two tab-housing assemblies such as the one defined, each assembly being located on a respective side of the callus remover. Preferably, the upper part comprises two lateral tabs, and the head comprises two housings.

In a possible embodiment, the head comprises securing means configured to contribute to securing the upper part in the closed position.

Preferably, the securing means comprise at least one hole-protuberance assembly, made up of: a hole and a protuberance, each one located in the upper part or the head, which are attached to one another to contribute to securing the upper part in the head. In a particular example, the callus remover comprises two hole-protuberance assemblies such as the one defined.

Preferably, the upper plate rotates with respect to the head to an upper position according to the usual grip in the usage position.

According to one embodiment, the callus remover comprises a recess through which an edge of the cutting blade emerges, the edge being intended for cutting calluses.

According to this embodiment, the cutting function is carried out by the lower part of the callus remover. In this case, the upper position is located in the upper part of the callus remover (which is opposite the lower part of the callus remover, where the cutting function is performed).

The callus remover with a safety fixing system that is described represents an innovation of hitherto unknown structural and constitutive features, reasons which, together with its practical usefulness, provide it with sufficient grounds to obtain the privilege of exclusivity being requested.

Particularly, the inventors find the manner of placing and removing the cutting blade in/from the callus remover to be advantageous, as compared to callus removers of the prior art.

In callus removers of the prior art, there is a risk of being cut with the cutting blade when handling the callus remover, as both hands are required to perform the operations of placing and removing the cutting blade, whereas in the callus remover object of the present invention the operations of placing and removing the cutting blade can be performed using only one hand, such that the other hand is free to handle the cutting blade, reducing the risk of the user being able to sustain accidental cuts.

A blade-securing plate with a mechanism which allows its rotation with respect to the head is included in other callus removers of the prior art. This plate rotates to the lower position according to the usual grip in the usage position, so, when opening the assembly formed by the blade-securing plate and the head, the cutting blade may fall due to gravity, as it is arranged below the head. However, in the callus remover object of the present invention, the upper plate rotates with respect to the head to the upper position according to the usual grip in the usage position, so, when opening the assembly formed by the upper plate and the head, there is no risk of the cutting blade falling, as is located above the head.

In other callus removers of the prior art including a protective cover for the cutting blade (function performed by the upper part of the present invention), when exerting force on the cover, it may be caused to move forward and come off, thereby leaving the cutting blade exposed, and, furthermore, there is a risk that it may fall out. However, the opposite occurs in the callus remover object of the present invention: In the usage position of the callus remover, the user exerts a forward force on the upper part, but the front stop limits the forward displacement. In this way, there is no risk of the upper part becoming detached, so the cutting blade is covered, and, furthermore there is no risk of it falling off.

The callus remover of the present invention improves the ease of the operation of changing and replacing the cutting blade.

In other callus removers of the prior art, the upper part is fully removable. However, in the callus remover object of the present invention, the upper part is attached to the callus remover even in the open position.

Herein, the word "comprises" and its variants must be interpreted as open expressions that do not intend to exclude the possibility of other technical features or components in addition to those explicitly mentioned. Furthermore, the word "comprises" includes the case of "consists of", which is interpreted as a closed expression that is limited to only the technical features or components that are explicitly mentioned. For those skilled in the art, other objects, advantages and features of the invention will be inferred in part from the description and in part from the practice of the invention. Furthermore, the present invention covers all the possible combinations of embodiments indicated herein.

### BRIEF DESCRIPTION OF THE DRAWINGS

To complement the description that is being made and for the purpose of helping to better understand the features of the invention, a set of drawings is attached as an integral part of said description in which the following is depicted in an illustrative and non-limiting manner:
Figure 1 shows an isometric view of the invention in the closed position.
Figure 2 shows an isometric view of the invention with the protrusions of the upper part located in the circular area of the corresponding slots.
Figure 3 shows an isometric view of the invention in the open position.
Figure 4 shows a detail of the displaceable hinge-type articulated attachment in a transition position between the open position and the closed position.
Figure 5 shows a detail of the displaceable hinge-type articulated attachment in the open position.
Figure 6 shows a top view of the invention in the closed position.
Figure 7 shows a top view of the invention with the protrusions of the upper part located in the circular area of the corresponding slots.
Figure 8 shows a top view of the invention in the open position.

List of reference numbers included in the figures:
1. Upper part
2. Head
3. Cutting blade
4. Displaceable hinge-type articulated attachment
5. Protrusions
6. Slots
7. Rectangular area of the slot
8. Circular area of the slot
9. Front stops
10. Holes
11. Protuberances
12. Assembly recesses
13. Lateral tabs
14. Housings
15. Rear stops
16. Handle

### EMBODIMENTS OF THE INVENTION

In the following detailed description of the preferred embodiments, reference is made to the attached drawings which are part of this specification, and specific preferred embodiments into which the invention can be carried out are shown in said drawings by way of illustration.

For the purpose of improving the understanding of the preferred embodiments, the closed position of the callus remover has been considered to coincide with the usage position, whereas the open position coincides with the position for accessing and taking out the cutting blade.

Namely, the present invention describe a callus remover with a safety fixing system comprising at least: a handle (16); a head (2) after the handle (16); an upper part (1); and a cutting blade (3) which is secured between the head (2) and the upper part (1); with the particularity that the fixing system has the following features:
The upper part (1) and the head (2) are attached by means of a displaceable hinge-type articulated attachment (4) which configures: a relative displacement of the upper part (1) with respect to the head (2); and a relative rotation between the upper part (1) and the head (2) in at least one position (synonymous with location), which is located along the relative displacement movement of the upper part (1) with respect to the head (2).

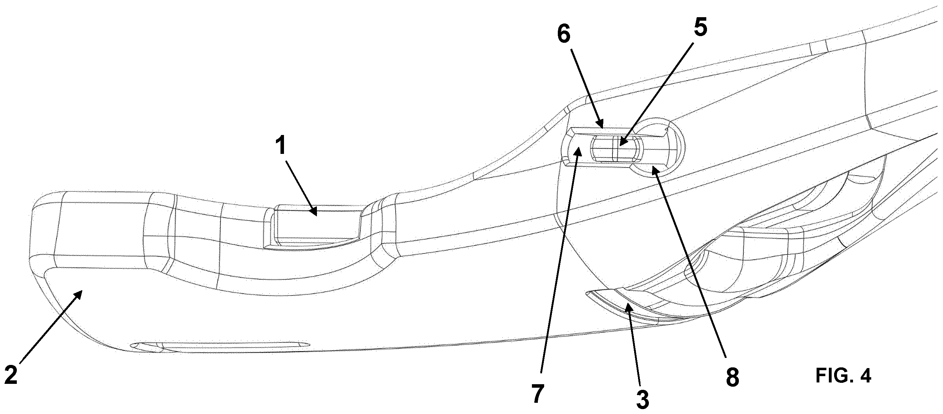

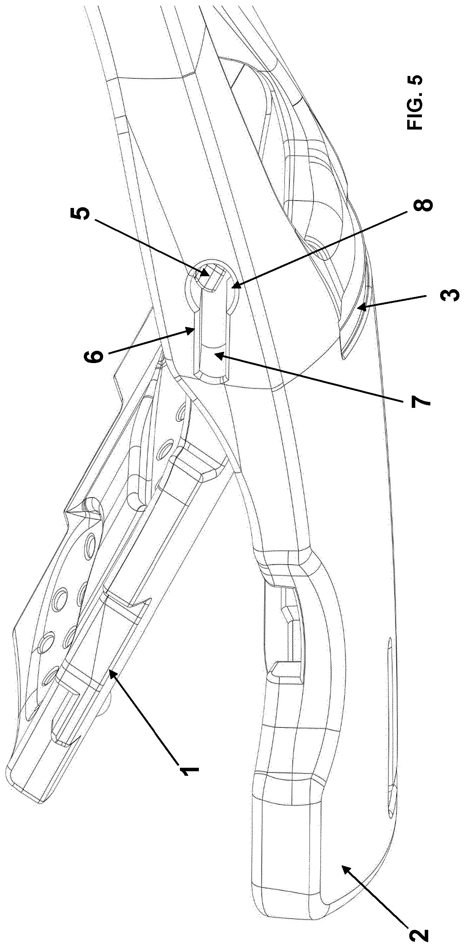

As can be observed in Figures 4 and 5, the displaceable hinge-type articulated attachment (4) comprises two protrusions (5) located on the sides of the upper part (1), each protrusion (5) on one side protruding from the upper part (1); and two slots (6) located in the head (2), each slot on one side of the head (2), with the protrusions (5) being at least partially inserted into the respective slots (6) such that:
- the protrusions (5), and therefore the upper part (1), can be displaced along the respective slots (6); and
- in at least one location of the protrusions (5) in the respective slots (6), rotation of the upper part (1) with respect to the head (2) is allowed.

According to a preferred embodiment:
- each slot (6) comprises a rectangular area (7) defined by two parallel planes separated from one another by a distance referred to as "slot thickness"; and a circular area (8), such that the slot thickness is smaller than the diameter of the circular area (8).
- each protrusion (5) is defined by two flat surfaces parallel to one another, and two curved surfaces serving as a lateral attachment between the parallel flat surfaces, such that the distance between the curved surfaces is greater than the distance between the parallel flat surfaces. The larger dimension of the protrusion (5) is referred to as the "length of the protrusion", and the smaller dimension of the protrusion (5) is referred to as the "thickness of the protrusion".

Notwithstanding the foregoing, the protrusions (5) could also have a rectangular prism shape, or similar geometries, even if the operation of the mechanism is not optimal.

Preferably:
- The thickness of the protrusion (5) coincides with the thickness of the rectangular area (7) of the slot (6). More specifically, the thickness of the protrusion (5) is smaller than the thickness of the rectangular area (7) of the slot (6), configuring a sufficient clearance so as to allow the sliding of the protrusion (5) along the slot (6), but insufficient to allow the rotation of the protrusion (5) inside the rectangular area (7) of the slot (6).
- The length of the protrusion (5) coincides with the diameter of the circular area (8) of the slot (6). More specifically, the length of the protrusion (5) is smaller than the diameter of the circular area (8) of the slot (6), configuring a sufficient clearance so as to allow the rotation of the protrusion (5) inside the circular area (8) of the slot (6), and, therefore, the rotation of the upper part (1) with respect to the head (2).

Preferably, when the callus remover is closed, the cutting blade (3) is curved between the upper part (1) and the head (2) such that the cutting blade (3) tends to recover its original flat shape; but the upper part (1) prevents this, so the cutting blade (3) generates a thrust on the upper part (1).

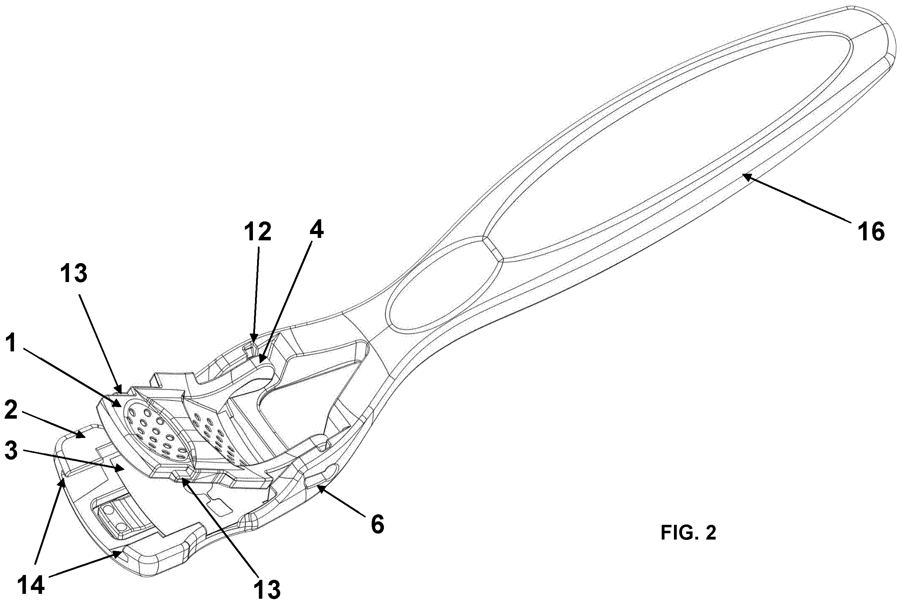

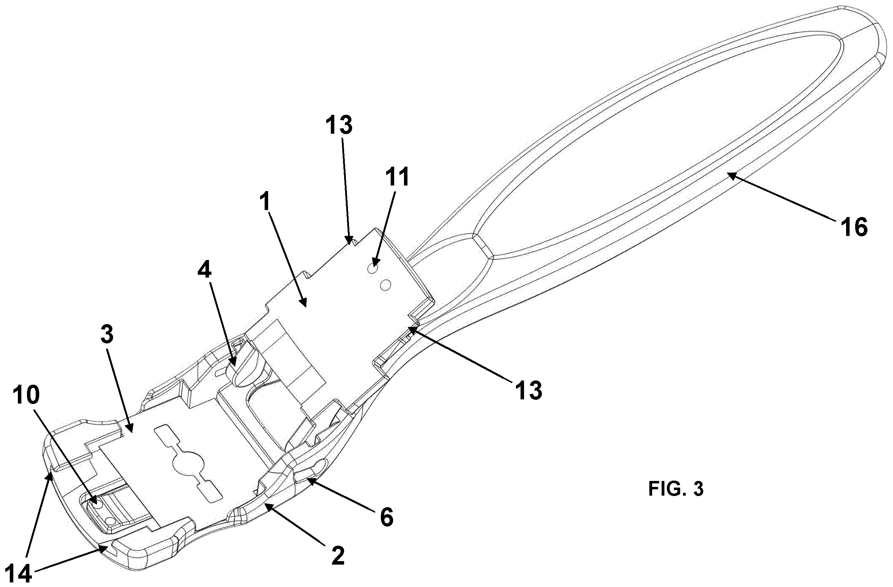

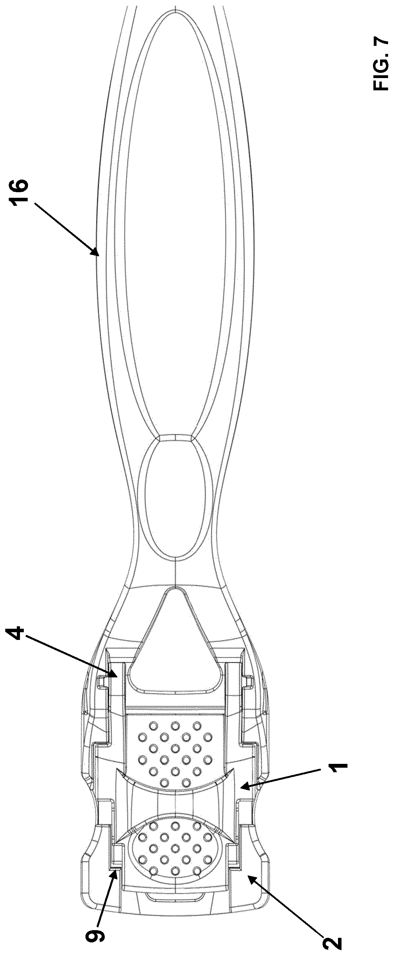

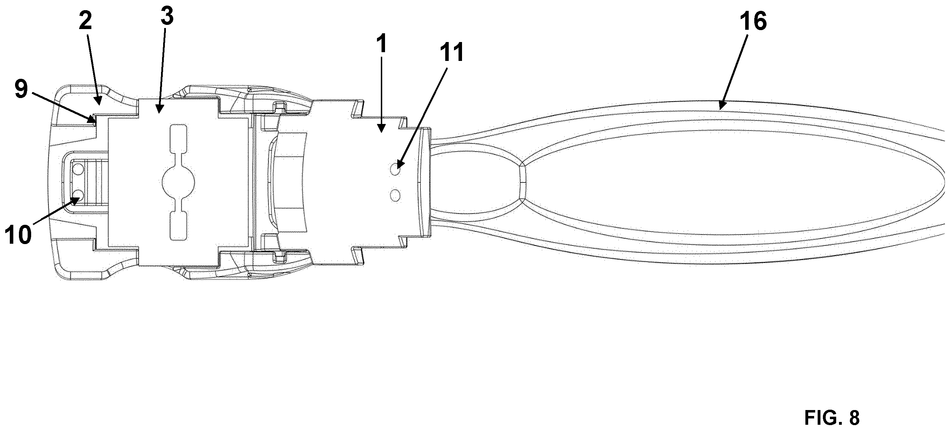

According to the above embodiments, to achieve the opening of the upper part (1) with respect to the head (2) according to Figures 3 and 8, all that is required is to press the upper part (1) against the head (2) and slide the upper part (1) to the position in which the protrusion (5) is located in the circular area (8) of the slot (6) of the head (2), as shown in Figure 2, and in more detail in Figure 5, allowing the relative rotation between the upper part (1) and the head (2), and therefore the release of the cutting blade (3). An open position of the upper part (1) with respect to the head (2) is thereby defined, as shown in Figure 7.

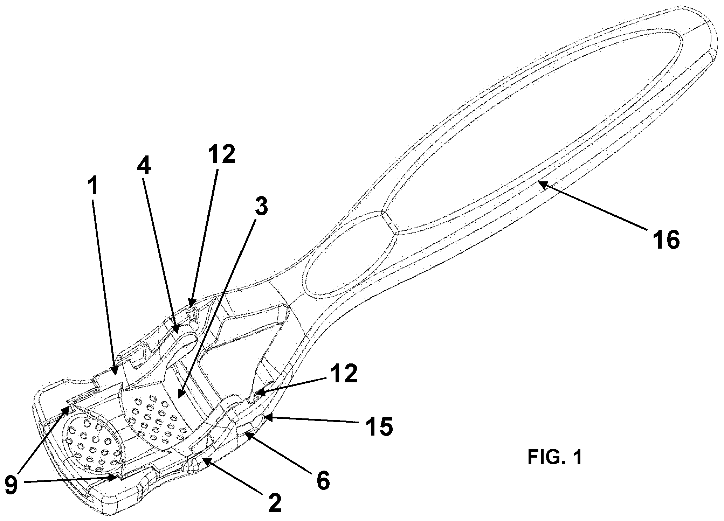

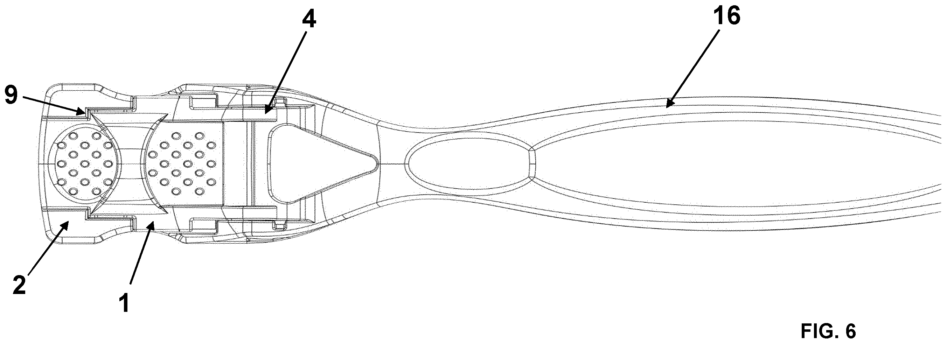

To close the upper part (1) and the head (2), housing a cutting blade (3) therein, requires maneuvers opposite the above maneuvers, i.e., the relative rotation of the upper part (1) in the head (2) until the protrusion (5) is aligned with the rectangular area (7) of the slot (6) of the head (2), and the sliding of the upper part (1) to a final fixing position. A closed position of the upper part (1) and the head (2) shown in Figures 1 and 6 is thereby defined.

A transition position, shown in Figure 4, in which the protrusion (5) of the upper part (1) is located in the rectangular area (7) of the slot (6) of the head (2), but still has not reached the final fixing position, is thereby defined between the open position of the upper part (1) with respect to the head (2), and the closed position of the upper part (1) and the head (2).

Preferably, the head (2) comprises stops for limiting the longitudinal displacement of the upper part (1) with respect to the head (2). According to a particular embodiment, the head (2) comprises front stops (9) and rear stops (15). The front stops (9) limit the forward displacement of the upper part (1), beyond the closed position. The rear stops (15) limit the backward movement of the upper part (1), beyond the open position.

According to a particular embodiment, the slots (6) themselves can act as elements for limiting the movement of the upper part (1), as the protrusions (5) can only be displaced along the length of the slots (6).

Preferably, the invention comprises securing means between the upper part (1) and the head (2) which contribute to securing the upper part (1) on the head (2) in the closed position of the callus remover.

The closed position, coinciding with a usage position of the callus remover, in which the upper part (1) is removably fixed to the head (2), and the open position in which the upper part (1) is released with respect to the head (2), are thereby configured .

Preferably, the securing means comprise at least one hole-protuberance assembly, made up of:
- a hole (10) in one of the following components: the upper part (1) or the head (2); and
- a protuberance (11) corresponding with the hole (10) and located in the component opposite the one in which the hole (10) is located.

According to the above embodiments, the combination of the hole (10) and the protuberance (11) contributes to the removable securing between the upper part (1) and the head (2) in the final fixing position, after sliding the upper part (1) along the rectangular area (7) of the slot (6) of the head (2).

According to a particular embodiment, the hole (10) or the protuberance (11) is arranged in a lug of the head (2). This lug swivels due to elasticity of the material and allows a safety clipping.

According to a particular embodiment, the securing means comprise two hole-protuberance assemblies such as those defined above.

Preferably, the invention comprises assembly recesses (12), configured to facilitate the assembly and/or disassembly of the upper part (1) with respect to the head (2).

As discussed above in relation to a particular embodiment, when the callus remover is closed, the cutting blade (3) is curved between the upper part (1) and the head (2) such that the cutting blade (3) tends to recover its original flat shape; but the upper part (1) prevents this, so the cutting blade (3) generates a thrust on the upper part (1). When the upper part (1) is slid to the open position, in which the protrusion (5) is located in the circular area (8) of the slot (6) of the head (2), allowing the relative rotation between the upper part (1) and the head (2), the thrust of the cutting blade (3), when it recovers its flat shape, causes the automatic opening of the upper part (1), releasing the cutting blade (3) for its replacement.

In a preferred embodiment, the upper part (1) comprises two lateral tabs (13), and the head (2) comprises two housings (14), such that, in the closed position of the upper part (1) and the head (2), the lateral tabs (13) are inserted into the respective housings (14).

The lateral tabs (13) and the housings (14) can perform the following function: by means of the thrust exerted by the cutting blade (3) on the upper part (1), friction is generated between the outer face of the lateral tabs (13) and the inner face of the housings (14), which ensures the closed position of the assembly, preventing a relative displacement between the upper part (1) and the head (2). According to another possible function, the lateral tabs (13) and the housings (14) contribute to preventing accidental rotation of the upper part (1) with respect to the head (2) from taking place.

The lateral tabs (13) are inserted into the housings (14) by means of the relative displacement of the upper part (1) with respect to the head (2).

Having sufficiently described the nature of the present invention, as well as the manner of putting it into practice, it is not considered necessary to further explain it so that any person skilled in the art can understand its scope and the advantages derived from it, it being noted that, within its essential nature, it may be put into practice in other embodiments that differ in detail from the embodiment indicated by way of example, and to which the protection that is sought will likewise be applied, provided that its fundamental principle is not altered, modified or changed.

## Claims

1. A callus remover with a safety fixing system, **characterized in that** it comprises at least:
a handle (16); a head (2) after the handle (16); an upper part (1); and a cutting blade (3) which is secured between the head (2) and the upper part (1); with the particularity that the safety fixing system has the following features: the upper part (1) and the head (2) are attached by means of a displaceable hinge-type articulated attachment (4) which configures:
- a relative displacement of the upper part (1) with respect to the head (2); and
- a relative rotation between the upper part (1) and the head (2) in at least one location included along the relative displacement of the upper part (1) with respect to the head (2).

2. The callus remover according to claim 1, **characterized in that** the displaceable hinge-type articulated attachment (4) comprises at least one movement means made up of the following components:
- a guiding component in the head (2), which allows the guided longitudinal displacement of the upper part (1) with respect to the head (2); and
- a guided component in the upper part (1), such that the guided component and, accordingly, the upper part (1), can be displaced in a guided manner along the head (2);
with the particularity that, in at least one location of the guided component in the guiding component, the guided component can rotate with respect to the guiding component, and, accordingly, the upper part (1) can rotate relatively with respect to the head (2).

3. The callus remover according to claim 2, **characterized in that** the displaceable hinge-type articulated attachment (4) comprises two movement means, one on each side of the callus remover.

4. The callus remover according to any of the preceding claims, **characterized in that** the guiding component is a slot (6) located longitudinally in the head (2); and the guided component is a protrusion (5) located in the upper part (1), which is at least partially inserted into the respective slot (6).

5. The callus remover according to claim 4, **characterized in that**:
- the slot (6) comprises two areas: a wide area and a narrow area; such that:
▪ the slot (6) has a substantially circular shape in the wide area;
▪ the narrow area of the slot (6) is defined between two parallel planes separated from one another by a distance referred to as "slot thickness", with the slot thickness being smaller than the recess of the wide area;
- the protrusion (5) has a substantially rectangular cross-section morphology, with a larger dimension referred to as "length of the protrusion" and a smaller dimension referred to as "thickness of the protrusion".

6. The callus remover according to claim 5, **characterized in that**:
- the thickness of the protrusion (5) is smaller than the thickness of the narrow area of the slot (6), configuring a sufficient clearance so as to allow the sliding of the protrusion (5) along the slot (6), but insufficient to allow the rotation of the protrusion (5) inside the narrow area of the slot (6);
- the length of the protrusion (5) is smaller than the recess of the wide area of the slot (6), configuring a sufficient clearance so as to allow the rotation of the protrusion (5) inside the wide area of the slot (6).

7. The callus remover according to claim 1, **characterized in that** the displaceable hinge-type articulated attachment (4) comprises: two protrusions (5) located on the sides of the upper part (1), with each protrusion (5) on one side protruding from the upper part (1); and two slots (6) located in the head (2), with each slot (6) on one side of the head (2), wherein:
- the protrusions (5) are at least partially inserted into the respective slots (6);
- the protrusions (5) can be displaced longitudinally along the respective slots (6); and
- rotation of the upper part (1) with respect to the head (2) is allowed in at least one position of the protrusions (5) in the respective slots (6).

8. The callus remover according to claim 7, **characterized in that**:
- each slot (6) comprises at least: a rectangular area (7) defined by two parallel planes separated from one another by a distance referred to as "slot thickness"; and a circular area (8), such that the slot thickness is smaller than the diameter of the circular area (8); and
- each protrusion (5) is defined by: two flat surfaces parallel to one another, and two curved surfaces which laterally attach the parallel flat surfaces to one another, such that the distance between the curved surfaces, referred to as "length of the protrusion", is greater than the distance between the parallel flat surfaces, referred to as "thickness of the protrusion".

9. The callus remover according to claim 8, **characterized in that**:
- the thickness of the protrusion (5) is smaller than the thickness of the rectangular area (7) of the slot (6), configuring a sufficient clearance so as to allow the sliding of the protrusion (5) along the slot (6), but insufficient to allow the rotation of the protrusion (5) inside the rectangular area (7) of the slot (6);
- the length of the protrusion (5) is smaller than the diameter of the circular area (8) of the slot (6), configuring a sufficient clearance so as to allow the rotation of the protrusion (5) inside the circular area (8) of the slot (6).

10. The callus remover according to any of the preceding claims, **characterized in that** the head (2) comprises stops limiting the longitudinal displacement of the upper part (1) with respect to the head (2).

11. The callus remover according to any of the preceding claims, **characterized in that** it comprises securing means between the upper part (1) and the head (2) which contribute to securing the upper part (1) on the head (2) in a closed position of the callus remover.

12. The callus remover according to claim 11, **characterized in that** the securing means comprise at least one hole-protuberance assembly made up of:
- a hole (10) in one of the following components: the upper part (1) or the head (2); and
- a protuberance (11) corresponding with the hole (10) and located in the component opposite the one in which the hole (10) is located.

13. The callus remover according to any of the preceding claims, **characterized in that** the cutting blade (3) has an original flat shape, but when the callus remover is closed, it is curved between the upper part (1) and the head (2), such that it generates a thrust on the upper part (1).

14. The callus remover according to any of the preceding claims, **characterized in that** it comprises at least one tab-housing assembly made up of:
- a tab (13) in one of the following components: the upper part (1) or the head (2); and
- a housing (14) in the component opposite the one in which the tab (13) is located; such that, in the closed position of the callus remover, the tab (13) is inserted into the housing (14).

15. The callus remover according to claim 14, **characterized in that** it comprises two tab-housing assemblies, with the particularity that the upper part (1) comprises two lateral tabs (13), and the head (2) comprises two housings (14), such that, in the closed position of the callus remover, the lateral tabs (13) are inserted into the respective housings (14).
